# EUROPEAN PATENT APPLICATION

(11) **EP 3 940 072 A1**
(43) Date of publication of application: **19.01.2022**
(21) Application number: 20185532.7
(22) Date of filing: 13.07.2020
(51) Int. Cl.: C12N 11/04, C12N 11/10, C12N 1/04, C12N 5/00, C12N 5/071, A61K 9/14, A61K 9/50, G01N 21/64

(54) **ALGINATE BEADS AND PRODUCTION THEREOF**

(71) Applicant: Biomillenia SAS, 93230 Romainville (FR)
(72) Inventor: DUBOYS, Thomas, 75011 Paris (FR); DU, Guansheng, 94200 IVRY SUR SEINE (FR); LOEFFERT, Dirk, 42781 Haan (DE); GRIFFITHS, Andrew, 75006 Paris (FR)
(74) Representative: CH Kilger Anwaltspartnerschaft mbB

(57) **Abstract**

The present invention aims to solve the problem by providing methods for producing solid, hollow and 2-layer alginate beads, wherein the alginate solidifies via slow release of divalent ions into an alginate solution. It is here provided a solution that allows a slow release of divalent cations into an alginate solution leading to solidification of the solution and formation of homogeneous beads, hollow beads or 2-layer beads. This invention also improves the viability of cells and microorganisms compared to other slow divalent cation release techniques to produce alginate beads or capsules.

Further, it provides a use of the methods for producing solid, hollow and 2-layer alginate beads for co-culturing fluorescent reporter cell-lines and microorganisms, wherein the fluorescent reporter cell-lines and the microorganisms are co-encapsulated into the core or shell of the alginate beads.

## Description

### FIELD OF THE INVENTION

The present invention is in the field of polymer chemistry and relates to methods for producing alginate beads. The present application is in the field of mammalian and microbial cell culture analysis and also, in the field of microfluidics, particularly in the field of microfluidic analysis and devices.

### BACKGROUND

Microfluidic devices are powerful tools that allow to miniaturise and to perform a large number of assays in parallel. As a consequence, microfluidic devices are ideal tools to vastly increase the throughput of many types of laboratory assays, such as screenings analyses or *in vitro* evolution.

Microfluidic devices are essentially networks of small channels used for the precise manipulation of small amounts of fluids. Miniaturised reaction vessels, which are in facts droplets of fluid, flow through the channels of the microfluidic system. Along their flow path, the droplets can be manipulated. A reagent can for example be added to at least a subset of the droplets by various methods known in the art, such as nanoinjection. This technique allows the production and precise manipulation of emulsion droplets revealing an enormous potential for high-throughput screening applications.

The assays used in such methods often require a precise incubation period after mixing of all of the reagents. Microfluidic is also used to produce beads of various compositions and sizes that have applications such as in ELISA assays, in attachment of nucleic acids for molecular biology assays or cell and microbial encapsulation. It has the advantage of producing extremely monodisperse emulsions with a controlled size that can then be solidified post emulsification via cross-linking of a polymer for example.

Alginate is a polymer extracted from brown algae that has been extensively used in biomedical applications, due to its non-toxicity. Applications include the encapsulation of molecules such as enzymes, proteins, antibodies, polymers, nucleic acids and/or probiotics for drug delivery or the encapsulation of microorganisms, cell lines or a combination of both for research purposes. For encapsulation, alginate is used in its hydrogel form. Gelling is obtained via cross-linking of the alginate polymers. This can be done via UV exposure or heating. However, these methods are harmful to the cells.

Alginate molecules are also cross-linked in the presence of divalent cations ions (ie. Ca²+, Ba²+). Therefore, when encapsulating cells or microorganisms this is preferably used to increase their viability. To obtain beads or capsules via divalent cationic cross-linking, two main techniques are used: 1) dripping of alginate containing the cells or microorganisms into a calcium bath or 2) slow release of calcium from a chelating agent such as EDTA. The first technique can lead to inhomogeneous particles particularly in the production of core-shell capsules smaller than 150 µm, due to a quick solidification process. These particles cannot be analyzed via standard flow cytometry instruments. The second technique leads to homogenous beads or core-shell capsules. However, it is harmful to the cells or microorganisms since the divalent cation is released either via a decrease in pH, or via competition with another ion. In both cases the chelating agents render nutrients from the surrounding environment unexploitable for the cells and microorganisms.

It would therefore be useful, to have a technique, with slow release of divalent cations into the alginate solution, which produces small homogeneous core-shell capsules and beads, with improved viability and less toxicity. There is therefore a need of producing alginate beads that solidify quickly and homogeneously while being less harmful to the cells/microorganisms in order to carry live cells.

### BRIEF DESCRIPTION OF THE INVENTION

The present invention provides a method for producing alginate beads, wherein the alginate solidifies via slow release of divalent ions into an alginate solution, comprising the steps of:
- preparing a first solution containing alginate;
- preparing a second solution containing an anionic surfactant in oil;
- preparing a third solution containing divalent ions;
- injecting said first and second solutions into a microfluidic device generating alginate in oil emulsions;
- collecting and incubating the alginate in oil emulsions in the third solution;
- inducing solidification of the first solution.

The present invention provides a method for producing alginate hollow beads, wherein the alginate solidifies via slow release of divalent ions into an alginate solution, comprising the steps of:
- preparing a first solution containing alginate, consisting of water in oil in alginate double emulsions;
- preparing a second solution containing an anionic surfactant in oil;
- preparing a third solution containing divalent ions;
- injecting said first and second solutions into a microfluidic device generating alginate in oil emulsions;
- collecting and incubating the alginate in oil emulsions in the third solution;
- inducing solidification of the first solution.

The present invention provides a method for producing two-layer alginate beads, wherein the alginate solidifies via slow release of divalent ions into an alginate solution, comprising the steps of:
- preparing a first solution containing alginate, consisting of a suspension of alginate beads in the alginate solution;
- preparing a second solution containing an anionic surfactant in oil;
- preparing a third solution containing divalent ions;
- injecting said first and second solutions into a microfluidic device generating alginate in oil emulsions;
- collecting and incubating the alginate in oil emulsions in the third solution;
- inducing solidification of the first solution.

It is here provided methods that allow a slow release of divalent cations into an alginate solution leading to gelling of the solution and formation of homogeneous beads, hollow beads and 2-layer beads. This invention also improves the viability of cells and microorganisms compared to other slow divalent cation release techniques to produce alginate beads, hollow beads or 2-layer beads. Divalent ions are selectively transported between a more concentrated aqueous solution to a less concentrated aqueous solution containing alginate. This solidifies the alginate and turns it into a hydrogel.

The present invention also provides a use of the method for producing alginate hollow beads, wherein the first solution containing alginate consists of water in oil in alginate double emulsions.

It provides a use of the method for producing 2-layer alginate beads, wherein the first solution containing alginate consists of a suspension of alginate beads in alginate solution

Further, it provides a use of the method for producing alginate beads for co-culturing fluorescent reporter cell-lines and microorganisms, wherein the fluorescent reporter cell-lines and the cells are co-encapsulated into the beads.

It provides a use of the method for producing alginate beads for co-culturing fluorescent reporter cell-lines and cells, wherein the fluorescent reporter cell-lines and the cells are co-encapsulated into the alginate beads.

Further, it provides a use of the method for producing hollow alginate beads for co-culturing fluorescent reporter cell-lines and microorganisms, wherein the fluorescent reporter cell-lines and the microorganisms are co-encapsulated into the core of hollow alginate beads or in the shell.

It provides a use of the method for producing hollow alginate beads for co-culturing fluorescent reporter cell-lines and cells, wherein the fluorescent reporter cell-lines and the cells are co-encapsulated into the core of the hollow alginate beads or in the shell.

Additionally, it provides a method for producing 2-layer alginate beads for co-culturing fluorescent reporter cell-lines and microorganisms, wherein the fluorescent reporter cell-lines and the microorganisms are co-encapsulated in the center or the outer layer of the 2-layer alginate beads.

It provides a method for producing 2-layer alginate beads for co-culturing fluorescent reporter cell-lines and cells, wherein the fluorescent reporter cell-lines and the cells are co-encapsulated in the center or the outer layer of the 2-layer alginate beads.

The methods here presented combine the advantages of two different solidification techniques which allow viability of cells and microorganisms of interests and homogeneity of the bead.

### DETAILED DESCRIPTION OF THE INVENTION

As used herein, the term "emulsion" refers to immiscible liquids that coexist in a mixture.

As used herein, the term "molecules" refers to enzymes, proteins, antibodies, polymers, nucleic acids that need to be encapsulated and/or analyzed.

As used herein, the terms "more concentrated" and "less concentrated" refer to divalent ions which are selectively transported between solutions of 1 mM to 10 M CaCl₂ to a solution with no CaCl₂.

As used herein, the term "micellar transport" refers to the transport of a chemical, between two solutions it is miscible in, through a solution it is not miscible in, by being enclosed in the center of a micelle composed of surfactant molecules.

As used herein, the term "double layer emulsion" refers to an emulsion within an emulsion.

As used herein, the term "bead" refers to a spherical solid object that remains stable in an aqueous solution.

As used herein, the term "hollow" refers to an object surrounding an empty space in its center.

As used herein, the term "droplet breaker" refers to demulsifiers to break the droplets, such as chloroform or 1H,1H,2H,2H-Perfluoro-1-octanol. These chemicals displace surfactants from the oil-water interfaces of the droplets and make the droplets unstable.

As used herein, the term "alginate" refers to a whole family of linear copolymers containing blocks of (1,4)-linked β-D-mannuronate (M) and α-L-guluronate (G) residues. The blocks are composed of consecutive G residues (GGGGGG), consecutive M residues (MMMMMM), and alternating M and G residues (GMGMGM).

As used herein, the term "cell medium" refers to a medium for culturing cells, a medium for increasing proliferation or differentiation.

As used herein, the term "microorganism" refers to bacteria, archaea, fungi, yeast, phages, viruses.

As used herein, the term "droplet" refers to a measure of volume and further relates to an isolated portion of a first fluid (first solution) that is surrounded by a second fluid (second solution).

As used herein, the terms "first", "second" and "third" solutions are used to discriminate solutions according to their content.

As used herein, the words "capsule" and "hollow bead" are used interchangeably.

As used herein, the term "two-layer bead" refers to a spherical object composed of a solid polymer bead in its center surrounded by a solid polymer layer.

As the method is performed in a microfluidic system, the term "droplet" also refers to "microfluidic droplet". Therefore, in the context of a microfluidic system, the term "droplet" also refers to an isolated portion of a "first" solution that is surrounded by a "second" solution, where the first and second solutions are immiscible.

As used herein, a "change in fluorescence" refers to a reduction or increase of the fluorescent signal produced by the reporter cell line. A reduction of the fluorescent signal refers to a situation where the fluorescence of the reporter cell line is reduced by a stimulus compared to the reporter cell in the exact same conditions minus the stimulus. An increase of the fluorescent signal refers to a situation where the fluorescence of the reporter cell line is increased by a stimulus compared to the reporter cell in the exact same conditions minus the stimulus.

As used herein, "activity on the fluorescent reporter cell line" refers to a situation where the microorganism modifies the fluorescent signal produced by the reporter cell by altering a biological pathway.

As used herein, "post-processing" refers to addition of droplets breaker and removal of remaining oil in the collection vial.

As used herein, a "dynamic droplet incubator " refers to a device allowing the increased diffusion of calcium to the droplets via cycling of the calcium loaded oil.

### The production of alginate beads

Alginate beads are produced using a microfluidic system in which, 2% w/v sodium alginate is cut by fluorinated oil (e.g. HFE7500) containing an anionic fluorinated surfactant producing alginate in oil emulsions and collected in a solution containing 200 mM CaCl₂. Calcium then diffuses from the collection solution to the emulsions through the oil. The calcium then cross-links the alginate, solidifying it and resulting in a hydrogel bead. These beads are collected and released from the oil envelope using perfluoro-1-octanol and resuspended in medium.

The present invention refers to a method for producing alginate beads, wherein the alginate solidifies via slow release of divalent ions into an alginate solution, comprising the steps of:
- preparing a first solution containing alginate;
- preparing a second solution containing an anionic surfactant in oil, wherein said second solution is immiscible with the first solution;
- preparing a third solution containing divalent ions;
- injecting said first and second solutions into a microfluidic device generating alginate in oil droplets;
- collecting and incubating the alginate in oil droplets in the third solution, inducing solidification of the first solution.

According to the stages or step of the method of the present invention, the droplets may be contained in a microfluidic system (on-chip) or in a collector device (off-chip) separate from the microfluidic system. The droplets may have a spherical or non-spherical form.

In one embodiment, the alginate beads are solid beads. In a preferred embodiment, the alginate beads are hollow alginate beads or 2-layer beads.

In one embodiment, the first solution comprises sodium alginate at 0.1% w/v to 20% w/v, more preferably 0.5 % w/v to 10% w/v, even more preferably 2% w/v to 4% w/v and most preferred 2% w/v.

In one embodiment, the second solution comprises a surfactant and a fluorinated oil. In one embodiment, the fluorinated oil is HFE7500 and the surfactant Krytox FSH. The w/v concentration of Krytox FSH in HFE7500 is defined. The concentration is between 0.01% w/w and 50% w/w, preferably between 1% w/w and 15% w/v, more preferably between 2.5% w/w and 12.5% w/w, even more preferably between 5% w/w and 10% w/w and most preferred 10% w/w.

In one embodiment the divalent ions are Mg²⁺, Zn²⁺, Hg²⁺, Cu²⁺, Sn²⁺, Fe²⁺, Co²⁺, Mn²⁺, Pb²⁺ Ca²⁺ and/or Ba²⁺. In a preferred embodiment the divalent ions are Ca²⁺ and/or Ba²⁺ and most preferred Ca²⁺.

In one embodiment, the incubation of the alginate in oil emulsions in the third solution containing CaCl₂ is performed for a period of 1 min to 30 days. In a preferred embodiment, for a period of 30 min to 24h. In a more preferred embodiment between 1h and 12h. In an even more preferred embodiment, between 2h and 4h and most preferably of 2h

In one embodiment, the concentration of the CaCl₂ solution is between 1 mM and 10 M, preferably between 10 mM and 1000 mM, more preferably between 100mM and 300mM, even more preferably between 150mM and 250mM and most preferably 200mM

In one embodiment to the alginate in oil emulsion in the third solution is added a droplet breaker, and wherein the droplet breaker is 1H,1H,2H,2H-Perfluoro-1-octanol.

In one embodiment, the solidification of the first solution is done via micellar transport of divalent ions from the third solution into the first solution via the second solution.

In one embodiment, the micellar transport of the calcium ions from the third solution to the first solution inducing the solidification is performed at a temperature between 1°C to 95°C, preferably between 15°C to 50°C, more preferably 17.5°C to 35°C, even more preferably 20°C to 30°C, even more preferably 22°C to 25°C and most preferably 22°C.

In one embodiment, the first solution comprises sodium alginate in cell culture medium and cells are resuspended in said first solution.

In one embodiment, the cells are from any adherent cell line. In a preferred embodiment, the cells are gut cells.

The present invention refers to the use of the method for producing alginate beads for co-culturing fluorescent reporter cell-lines and microorganisms, wherein the cell-lines and microorganisms are co-encapsulated.

In one embodiment, the fluorescent reporter cell-line is encapsulated in the solid alginate bead.

In one embodiment the microorganism is tested for activity on the fluorescent reporter cell line.

In one embodiment, the fluorescent reporter cell-line is analyzed for a change in fluorescence.

In one embodiment, the first solution comprises sodium alginate in cell culture medium and cells are resuspended in said first solution.

In one embodiment, the cells are from any adherent cell line. In a preferred embodiment, the cells are gut cells.

Further, it provides a use of the method for producing alginate beads for co-culturing fluorescent reporter cell-lines and microorganisms, wherein the fluorescent reporter cell-lines and microorganisms are co-encapsulated into the beads.

The embodiments discussed herein with reference to method for producing alginate beads apply also to the use of the methods for producing alginate beads for co-culturing fluorescent reporter cell-lines and microorganisms.

Further, it provides a use of the method for producing alginate beads for co-culturing fluorescent reporter cell-lines and cells, wherein the fluorescent reporter cell-lines and cells are co-encapsulated into the beads.

The embodiments discussed herein with reference to method for producing alginate beads apply also to the use of the methods for producing alginate beads for co-culturing fluorescent reporter cell-lines and cells.

### The production of alginate hollow beads

Alginate hollow beads are produced using a succession of microfluidic systems to first produce triple emulsions. An aqueous solution is cut by a fluorinated oil in a microfluidic system producing simple emulsions. The single emulsions are reinjected in a second microfluidic system and cut by 2% w/v sodium alginate producing double emulsions. The double emulsions are reinjected into a third microfluidic system and cut by fluorinated oil (e.g. HFE7500) containing an anionic fluorinated surfactant producing water in oil in alginate in oil triple emulsions. The alginate in the triple emulsions then needs to be solidified. The triple emulsions are collected in a solution containing 200 mM CaCl₂. Calcium then diffuses from the collection solution to the alginate through the outer oil layer. The calcium cross-links the alginate, solidifying it and resulting in a hollow bead. These capsules are then released from the oil shell using perfluoro-1-octanol and resuspended in medium.

A further aspect of the present invention refers to a method for producing hollow alginate beads, wherein the alginate solidifies via slow release of divalent ions into an alginate solution, comprising the steps of:
- preparing a first solution containing alginate, consisting of water in oil in alginate double emulsions;
- preparing a second solution containing an anionic surfactant in oil;
- preparing a third solution containing divalent ions;
- injecting said first and second solutions into a microfluidic device generating alginate in oil emulsions;
- collecting and incubating the alginate in oil emulsions in the third solution,
   inducing solidification of the first solution.

In one embodiment, the first solution comprises sodium alginate at 0.1% w/v to 20% w/v, more preferably 0.5 % w/v to 10% w/v, even more preferably 2% w/v to 4% w/v and most preferred 2% w/v.

In one embodiment, the second solution comprises a surfactant and a fluorinated oil. In one embodiment, the fluorinated oil is HFE7500 and the surfactant Krytox FSH. The w/v concentration of Krytox FSH in HFE7500 is defined. The concentration is between 0.01% w/w and 50% w/w, preferably between 1% w/w and 15% w/v, more preferably between 2.5% w/w and 12.5% w/w, even more preferably between 5% w/w and 10% w/w and most preferred 10% w/w.

In one embodiment the divalent ions are Mg²⁺, Zn²⁺, Hg²⁺, Cu²⁺, Sn²⁺, Fe²⁺, Co²⁺, Mn²⁺, Pb²⁺ Ca²⁺ and/or Ba²⁺. In a preferred embodiment the divalent ions are Ca²⁺ and/or Ba²⁺ and most preferred Ca²⁺.

In one embodiment, the incubation of the alginate in oil emulsions in the third solution containing CaCl₂ is performed for a period of 1 min to 30 days. In a preferred embodiment, for a period of 30 min to 24h. In a more preferred embodiment between 1h and 12h. In an even more preferred embodiment, between 2h and 4h and most preferably of 2h

In one embodiment, the concentration of the CaCl₂ solution is between 1 mM and 100 M, preferably between 10 mM and 1000 mM, more preferably between 100mM and 300mM, even more preferably between 150mM and 250mM and most preferably 200mM

In one embodiment to the alginate in oil emulsion in the third solution is added a droplet breaker, and wherein the droplet breaker is 1H,1H,2H,2H-Perfluoro-1-octanol.

In one embodiment, the solidification of the first solution is done via micellar transport of divalent ions from the third solution into the first solution via the second solution.

In one embodiment, the micellar transport of the calcium ions from the third solution to the first solution inducing the solidification is performed at a temperature between 1°C to 95°C, preferably between 15°C to 50°C, more preferably 17.5°C to 35°C, even more preferably 20°C to 30°C, even more preferably 22°C to 25°C and most preferred 22°C.

In one embodiment, the fluorescent reporter cell-line is encapsulated in the hollow alginate bead.

In one embodiment, the fluorescent reporter cell-line is encapsulated in the core of the hollow alginate bead.

In one embodiment, the microorganism is encapsulated in the shell of the hollow alginate bead.

In one embodiment, the microorganism is encapsulated in the core of the hollow alginate bead.

In one embodiment the microorganism is tested for activity on the fluorescent reporter cell line.

In one embodiment, the fluorescent reporter cell-line is analyzed for a change in fluorescence.

In one embodiment, the first solution comprises sodium alginate in cell culture medium and cells are resuspended in said first solution.

In one embodiment, the cells are from any adherent cell line. In a preferred embodiment, the cells are gut cells.

Further, it provides a use of the method for producing hollow alginate beads for co-culturing fluorescent reporter cell-lines and microorganisms, wherein the fluorescent reporter cell-lines and microorganisms are co-encapsulated into the core of the hollow alginate beads or in the shell.

The embodiments discussed herein with reference to method for producing hollow alginate beads apply also to the use of the methods for producing hollow alginate beads for co-culturing fluorescent reporter cell-lines and microorganisms.

Further, it provides a use of the method for producing hollow alginate beads for co-culturing fluorescent reporter cell-lines and cells, wherein the fluorescent reporter cell-lines and cells are co-encapsulated into the core of the hollow alginate beads or in the shell.

The embodiments discussed herein with reference to method for producing hollow alginate beads apply also to the use of the methods for producing alginate beads for co-culturing fluorescent reporter cell-lines and cells.

### The production of two-layer alginate beads

Alginate 2-layer beads are produced using a microfluidic system in which, 2% w/v sodium alginate is cut by fluorinated oil (e.g. HFE7500) containing an anionic fluorinated surfactant producing alginate in oil emulsions and incubated in a dynamic droplet incubator that cycles calcium loaded oil to the droplets. Calcium then diffuses from the oil to the emulsions through the oil. The calcium then cross-links the alginate, solidifying it and resulting in a hydrogel bead. These beads are collected and released from the oil shell using perfluoro-1-octanol and resuspended in 2% w/v sodium alginate solution. The alginate beads in alginate solution suspension is then is cut by fluorinated oil (e.g. HFE7500) containing an anionic fluorinated surfactant in a microfluidic device producing alginate beads in alginate solution in oil emulsions and incubated in a dynamic droplet incubator that cycles calcium loaded oil to the droplets. Calcium then diffuses from the oil to the emulsions through the oil. The calcium then cross-links the alginate, solidifying it and resulting in a 2-layer hydrogel bead. These beads are collected and released from the oil shell using perfluoro-1-octanol and resuspended.

A further aspect of the present invention refers to a method for producing 2-layer alginate beads, wherein the alginate solidifies via slow release of divalent ions into an alginate solution, comprising the steps of:
- preparing a first solution containing alginate, consisting of a suspension of alginate beads in an alginate solution;
- preparing a second solution containing an anionic surfactant in oil;
- preparing a third solution containing divalent ions;
- injecting said first and second solutions into a microfluidic device generating alginate in oil emulsions;
- collecting and incubating the alginate in oil emulsions in the third solution;
   inducing solidification of the first solution.

In one embodiment, the first solution comprises sodium alginate at 0.1% w/v to 20% w/v, more preferably 0.5 % w/v to 10% w/v, even more preferably 2% w/v to 4% w/v and most preferred 2% w/v.

In one embodiment, the second solution comprises a surfactant and a fluorinated oil. In one embodiment, the fluorinated oil is HFE7500 and the surfactant Krytox FSH. The w/v concentration of Krytox FSH in HFE7500 is defined. The concentration is between 0.01% w/w and 50% w/w, preferably between 1% w/w and 15% w/v, more preferably between 2.5% w/w and 12.5% w/w, even more preferably between 5% w/w and 10% w/w and most preferably 10% w/w.

In one embodiment the divalent ions are Mg²⁺, Zn²⁺, Hg²⁺, Cu²⁺, Sn²⁺, Fe²⁺, Co²⁺, Mn²⁺, Pb²⁺ Ca²⁺ and/or Ba²⁺. In a preferred embodiment the divalent ions are Ca²⁺ and/or Ba²⁺ and most preferably Ca²⁺.

In one embodiment, the incubation of the alginate in oil emulsions in the third solution containing CaCl₂ is performed for a period of 1 min to 30 days. In a preferred embodiment, for a period of 30 min to 24h. In a more preferred embodiment between 1h and 12h. In an even more preferred embodiment, between 2h and 4h and most preferably of 2h.

In one embodiment to the alginate in oil emulsion in the third solution is added a droplet breaker, and wherein the droplet breaker is 1H,1H,2H,2H-Perfluoro-1-octanol.

In one embodiment, the solidification of the first solution is done via micellar transport of divalent ions from the third solution into the first solution via the second solution.

In one embodiment, the micellar transport of the calcium ions from the third solution to the first solution inducing the solidification is performed at a temperature between 1°C to 95°C, preferably between 15°C to 50°C, more preferably 17.5°C to 35°C, even more preferably 20°C to 30°C, even more preferably 22°C to 25°C and most preferred 22°C.

In one embodiment, the fluorescent reporter cell-line is encapsulated in the 2-layer alginate bead.

In one embodiment, the fluorescent reporter cell-line is encapsulated in the center of the 2-layer alginate bead.

In one embodiment, the fluorescent reporter cell-line is encapsulated in the outer layer of the 2-layer alginate bead.

In one embodiment, the microorganism is encapsulated in the 2-layer alginate bead.

In one embodiment, the microorganism is tested for activity on the fluorescent reporter cell line.

In one embodiment, the fluorescent reporter cell-line is analyzed for a change in fluorescence.

In one embodiment, the first solution comprises sodium alginate in cell culture medium and cells are resuspended in said first solution.

In one embodiment, the cells are from any adherent cell line. In a preferred embodiment, the cells are gut cells.

Further, it provides a use of the method for producing 2-layer alginate beads for co-culturing fluorescent reporter cell-lines and microorganisms, wherein the fluorescent reporter cell-lines and microorganisms are co-encapsulated into in the center or in the outer layer of the 2-layer alginate beads.

The embodiments discussed herein with reference to method for producing 2-layer alginate beads apply also to the use of the methods for producing 2-layer alginate beads for co-culturing fluorescent reporter cell-lines and microorganisms.

Further, it provides a use of the method for producing 2-layer alginate beads for co-culturing fluorescent reporter cell-lines and cells, wherein the fluorescent reporter cell-lines and cells are co-encapsulated into in the center or in the outer layer of the 2-layer alginate beads.

The embodiments discussed herein with reference to method for producing 2-layer alginate beads apply also to the use of the methods for producing 2-layer alginate beads for co-culturing fluorescent reporter cell-lines and cells.

### Microfluidics

The experimentation is based on the key feature of microfluidic droplet systems that is the use of water-in-oil emulsion droplets to compartmentalize reagents into nanoliter to picolitre volumes. Droplet-based microfluidics manipulate discrete volumes of fluids in immiscible phases with low Reynolds number and laminar flow regimes. Two immiscible phases used for the droplet generation are referred to as the continuous phase (medium in which droplets are generated) and dispersed phase (the droplet phase). The size of the generated droplets is mainly controlled by the flow rates of the continuous phase and dispersed phase, interfacial tension between two phases and the geometry used for the droplet generation. The oil that separates the aqueous phase droplets can prevent cross-contamination between reagents in neighboring droplets and reduce the nonspecific binding between channel surface and the reagents. The same technical principles for fluid control and monitoring of droplets using fluorescence microscope were employed.

### EXAMPLES

### Example 1

### Preparation of alginate beads via micellar transport of calcium to the alginate through on oil phase with Krytox FSH in a tube

### Fabrication of master mold

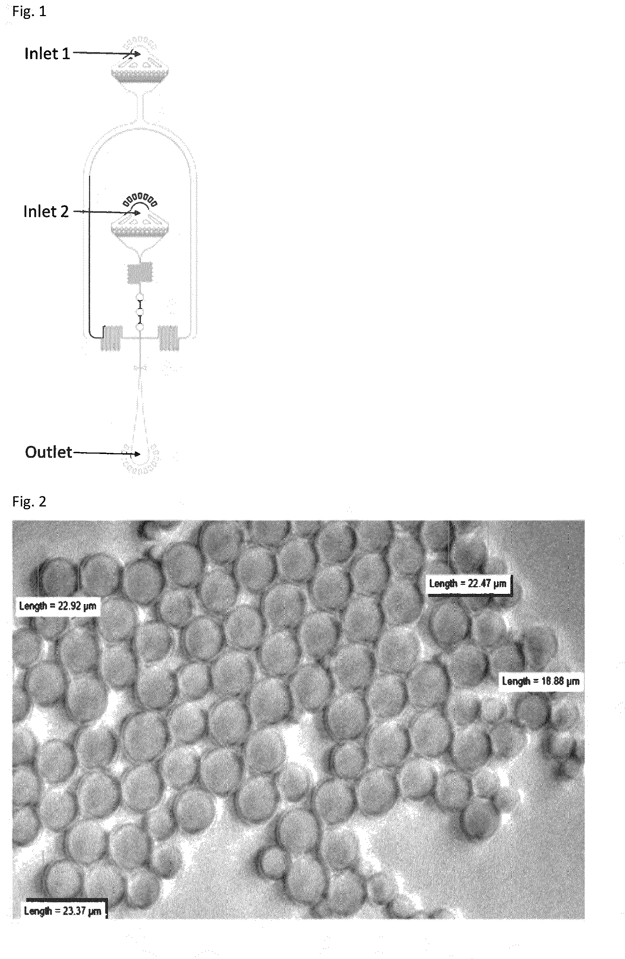

A master mold of 21µm in height is fabricated for preparing a microfluidic flow-focusing device (FFD) (Fig. 1).

A glass wafer was heated at 200°C for 5 min. Approximatively 5 mL of SU8- 2025 were spin coated at 500 rpm for 5 sec and at 3500rpm for another 30 sec; it was then heated at 65°C for 3min and at 95°C for 5min. The coated wafer was then exposed to 365 nm wavelength UV light at 21.1mW/cm2 through a quartz slide flush with the mask design for the flow-focusing device. The wafer was then heated at 65°C for 1min and at 95°C for 5min. The glass wafer was incubated in propylene glycol methyl ether acetate (PGMEA) in a beaker for 4min while rotating at 200rpm. The glass wafer was then cleaned with isopropanol and dried with an air gun; the glass wafer was heated again at 200°C for 5 min.

### Fabrication of flow-focusing devices (FFD) (fig. 1)

The master mold was placed in the center of an aluminum disc and the edges that were not covered by the master mold folded up like a tart dish. 50 g of polydimethylsiloxane (PDMS) were poured into a plastic disc. 5g of cross-linker were poured into the plastic dish with PDMS and mixed thoroughly. The mix was poured onto the master mold and placed in a vacuum chamber until bubbles were removed and then placed in a 70°C incubator for at least 4 hours. The master mold was removed from the incubator, the aluminum was peeled off from the master mold. Cross-linked PDMS was peeled off the master mold. PDMS was cut around the design of the microfluidic chip. 0.75 or 1 mm holes in inlets 1 and 2 and the outlet were punctured. The prepared PDMS slab and a glass slide were cleaned using isopropanol and dried using an air gun.

The PDMS and glass slide were activated in a plasma cleaner and the two pieces were bound together by making sure to remove any air pockets between them and incubated at 90°C for 5 min.

### Wettability modification of FFDs

1 mL of 1% w/w trichloro(1H,1H,2H,2H-perfluorooctyl)silane in HFE7500 was prepared. The FFD channel was filled from outlet with 1% w/w trichloro(1H,1H,2H,2H-perfluorooctyl)silane in HFE7500. The solution was then flushed out from inlet 2 using N₂ gas.

### Bead making

Solutions of 2% w/v sodium alginate (first solution), 10% w/w Krytox FSH, HFE7500 (second solution) and 200mM CaCl₂ (third solution) were prepared.

The molecule or organism of interest was added into the first solution and mixed. The first solution is loaded into the inlet 1 of the FFD microfluidic device (fig. 1) from a pressurized reservoir through PTFE (Polytetrafluoroethylene) tubing driven by a pressure pump using compressed air. The second solution was loaded into inlet 2 of the FFD microfluidic device also from a pressurized reservoir through PTFE tubing driven by a pressure pump using compressed air. Flowrates of the first and second solutions are adjusted in order to let the second solution cut the first solution at the T-junction in order to generate monodisperse droplets of suitable size depending on the application of the method.

### Solidification of alginate beads in a tube

Droplets were collected in 300 µL of the third solution in a 1.5 mL microfuge tube and incubated in the third solution for 2 hours with the microfuge tube in horizontal position.

### Resuspend the alginate emulsions in solution

100 µL of 1H,1H,2H,2H-Perfluoro-1-octanol were added to the collection tube, the solution mixed by inverting multiple times and briefly spun down using a tabletop centrifuge. Infranatant and supernatant were removed without disturbing beads and discarded. The alginate beads were resuspended in appropriate solution depending on application of the method.

### Generated beads

Following this protocol alginate beads were prepared via micellar transport of Ca²⁺ form the collection solution to alginate.

### Example 2:

### Solidification of alginate beads via micellar transport of calcium to the alginate through on oil phase with Krytox FSH in wet box

The time to solidify alginate droplets was assessed via diffusion of calcium through an oil layer in micelles of surfactant from a solution containing 200 mM CaCl₂ to alginate in HFE7500 droplets.

### Fabrication of flow-focusing devices (FFD) (Fig. 1))

Poly-(dimethylsiloxane) (PDMS) microfluidic FFDs were fabricated from master molds. The molds were produced by photolithography technique as described in example 1.

The master mold was placed in the center of an aluminum disc and the edges that were not covered by the master mold folded up like a tart dish. 50 g of polydimethylsiloxane (PDMS) were poured into a plastic disc. 5g of cross-linker were poured into the plastic dish with PDMS and mixed thoroughly. The mix was poured onto the master mold and placed in a vacuum chamber until bubbles were removed and then placed in a 70°C incubator for at least 4 hours. The master mold was removed from the incubator, the aluminum was peeled off from the master mold. Cross-linked PDMS was peeled off the master mold. PDMS was cut around the design of the microfluidic chip. 0.75 or 1 mm holes in inlets 1 and 2 and the outlet were punctured. The prepared PDMS slab and a glass slide were cleaned using isopropanol and dried using an air gun.

The PDMS and glass slide were activated in a plasma cleaner and the two pieces were bound together by making sure to remove any air pockets between them and incubated at 90°C for 5 min.

### Wettability modification of FFDs

1 mL of 1% w/w trichloro(1H,1H,2H,2H-perfluorooctyl)silane in HFE7500 was prepared. The FFD channel was filled from outlet with 1% w/w trichloro(1H,1H,2H,2H-perfluorooctyl)silane in HFE7500. The solution was then flushed out from inlet 2 using N₂ gas.

### Droplet generation

Solutions of 2% w/v sodium alginate (first solution), 10% w/w Krytox FSH, HFE7500 (second solution) and 200mM CaCl2 (third solution) were prepared.

Droplets of 2% w/v sodium alginate, MilliQ in 10% w/w Krytox FSH, HFE7500 were produced by flowing the alginate solution (first solution) at 1500 mbar into the FFD and injecting the HFE7500 solution (second solution) at 1500 mbar into the FFD and therefore cutting the alginate flow with the solution of HFE7500 at the flow-focusing area. The droplets were then collected in a 1.5 mL microfuge tube in 200 mM CaCl₂.

### Preparation and setup of wetbox

2 or 3 sheets of Kimtech wipes were soaked with MilliQ water until water dripped from them. They were then used to line the outskirts and the bottom of 5 petri dishes (wet boxes). 5x10 µL of 200 mM CaCl₂ were added to different areas of the petri dishes, forming droplets.

2 µL of collected droplets were deposited into the centre of the CaCl₂ droplets and then observed under the bright field filter of a fluorescence microscope.

2 µL of 1H,1H,2H,2H-Perfluoro-1-octanol (PFO) was added to various droplets of one wetbox and the process observed under the bright field filter of the fluorescence microscope.

The other plates were then incubated respectively 1h, 2h, 4h or overnight and again observed under the bright filed filter of the fluorescence microscope.

After incubation of the plates for 1h, 2h, 4h or overnight, 2 µL of PFO was added to various droplets and the droplets observed under the bright field filter of the fluorescence microscope.

### Generated droplets

Fig. 2 shows that the droplets are relatively polydisperse but the majority are approximatively between 18 and 23 µm. Better control of the droplet generation is obtained via using a droplet dye and monitoring the droplet size in combination with flow control, in order to obtain monodisperse droplets.

### Droplets after incubation and after addition of droplet breaker

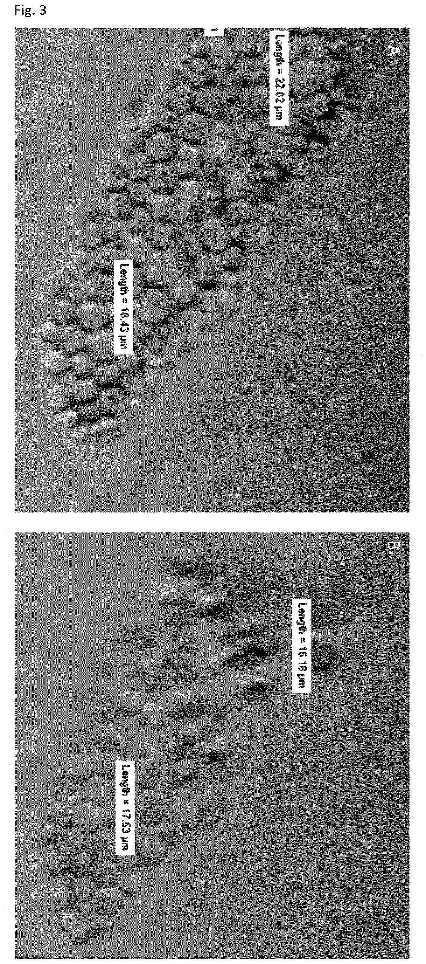

After 1 hour of incubation, the droplets slightly shrunk in size (fig. 3A). Upon addition of PFO the droplets are solid (fig. 3B). However, the produced droplets are polydisperse which makes it difficult to determine if the solidification occurs upon contact with the continuous phase or via micellar transport. In fig. 3B, the resuspended droplets are not homogeneous and non-spherical. Thus, the droplets solidify upon contact with the continuous phase. Inhomogeneous beads are an indication of fast solidification.

Fig. 4A compared to fig. 3A, shows that the droplets have significantly shrunk in size. This is due to osmosis. The continuous phase has a high concentration of CaCl₂, whereas the droplets do not contain CaCl₂. A buffer solution with various salts concentration not containing divalent ions could be used to counteract this effect.

Fig. 4B (after addition of PFO) shows that the droplets are homogeneous and spherical. Thus, the droplets solidify slowly and via micellar transport of CaCl₂.

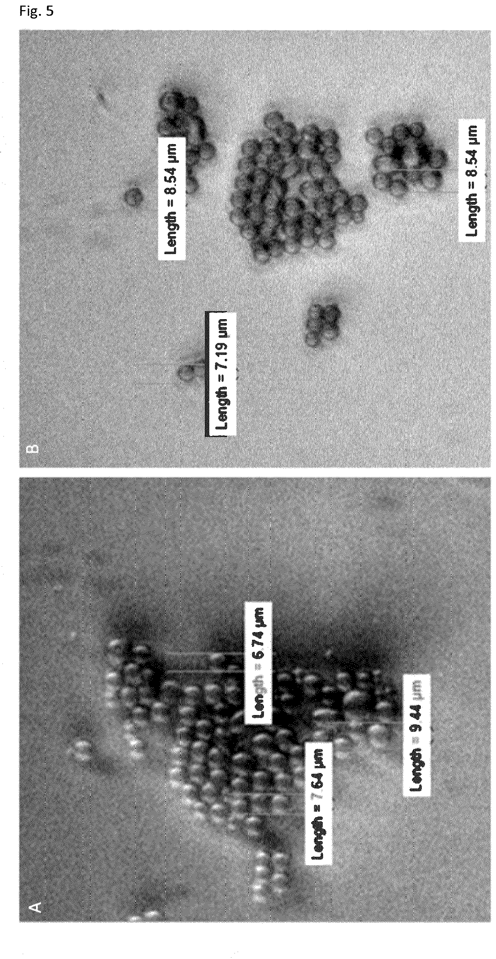

After 4 hours of incubation there is also shrinkage of the droplets (fig. 5A and 5B); However, not as significant as between 1- and 2-hours incubation.

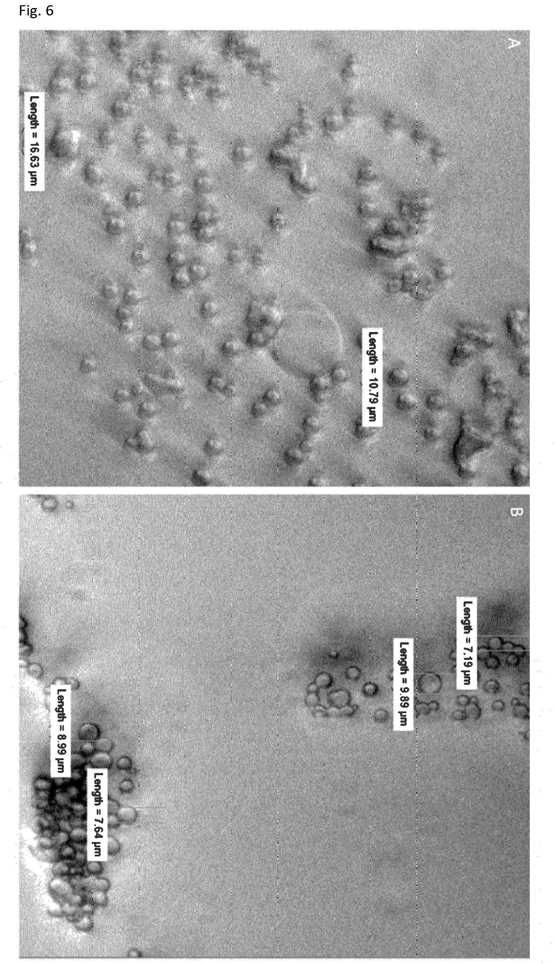

When adding PFO after 4- and 24 -hours incubation, similarly to the 2 hours incubation, the resuspended droplets are spherical and homogeneous (fig. 5B, fig. 6B). As described before, the droplets solidify via micellar transport of CaCl₂.

To conclude, it is possible to observe that the alginate beads solidify in 2 hours or more via micellar transport of CaCl₂.

### Example 3

### Solidifying alginate emulsions via calcium diffusion in swollen micelles with anionic fluorosurfactant

The solidification of the alginate droplets was assessed via diffusion of calcium through an oil layer in micelles of anionic fluorosurfactant.

### Fabrication of flow-focusing devices (FFD) (Fig. 1))

Poly-(dimethylsiloxane) (PDMS) microfluidic FFDs were fabricated from master molds. The molds were produced by photolithography technique as described in example 1.

The master mold was placed in the center of an aluminum disc and the edges that were not covered by the master mold folded up like a tart dish. 50 g of polydimethylsiloxane (PDMS) were poured into a plastic disc. 5g of cross-linker were poured into the plastic dish with PDMS and mixed thoroughly. The mix was poured onto the master mold and placed in a vacuum chamber until bubbles were removed and then placed in a 70°C incubator for at least 4 hours. The master mold was removed from the incubator, the aluminum was peeled off from the master mold. Cross-linked PDMS was peeled off the master mold. PDMS was cut around the design of the microfluidic chip. 0.75 or 1 mm holes in inlets 1 and 2 and the outlet were punctured. The prepared PDMS slab and a glass slide were cleaned using isopropanol and dried using an air gun.

The PDMS and glass slide were activated in a plasma cleaner and the two pieces were bound together by making sure to remove any air pockets between them and incubated at 90°C for 5 min.

### Wettability modification of FFDs

1 mL of 1% w/w trichloro(1H,1H,2H,2H-perfluorooctyl)silane in HFE7500 was prepared. The FFD channel was filled from outlet with 1% w/w trichloro(1H,1H,2H,2H-perfluorooctyl)silane in HFE7500. The solution was then flushed out from inlet 2 using N₂ gas.

### Droplet generation

Solutions of 2% w/v sodium alginate, MilliQ; 10% w/w Krytox, HFE7500 and 200 mM CaCl₂ were prepared.

Droplets of 2% w/v sodium alginate, MilliQ in 10% w/w Krytox, HFE7500 were prepared by flowing the alginate solution at 1250 mbar into the droplet making chip and injecting the HFE7500 solution at 650 mbar into the FFD. Therefore, cutting the alginate flow with the solution of HFE7500 at the flow-focusing area.

The droplets were then collected in a 1.5 mL microfuge tube in 300 µL 200 mM CaCl₂.

### Preparation, setup and observation of "wetbox"

2 or 3 sheets of Kimtech wipes were soaked in MilliQ water until no more water could be retained by the wipe. They were then used to line the outskirts of the bottom part of the petri dish (wetbox). 5x10 µL of 200 mM CaCl₂ were added to different areas of 2 "wetboxes", forming droplets.

2 µL of collected emulsions prepared using Krytox FSH were deposited into the centre of the CaCl₂ droplets, then observed under the bright field filter of the fluorescence microscope.

1 µL of 1H,1H,2H,2H-Perfluoro-1-octanol (PFO) was then added to various droplets of one of the petri dishes and the droplets under the bright field filter of the fluorescence microscope. The other petri dish was incubated overnight and again observed under the bright filed filter of the fluorescence microscope. After incubation 1 µL of PFO was added to various droplets of the petri dishes and the process observed under the bright field filter of the fluorescence microscope.

### Bulk experiment

In parallel to the "wetbox" the collection tube was also incubated overnight. 2 µL of emulsions were observed under the bright field filter of the fluorescence microscope before and after addition of 300 µL PFO and removal of oil from the vial.

### Droplet making

Krytox FSH seems to completely dissolve in HFE7500. Droplets were successfully prepared and were stable until the outlet. Krytox can be used as a surfactant to prepare water in HFE7500 emulsions.

### Droplets after droplet generation

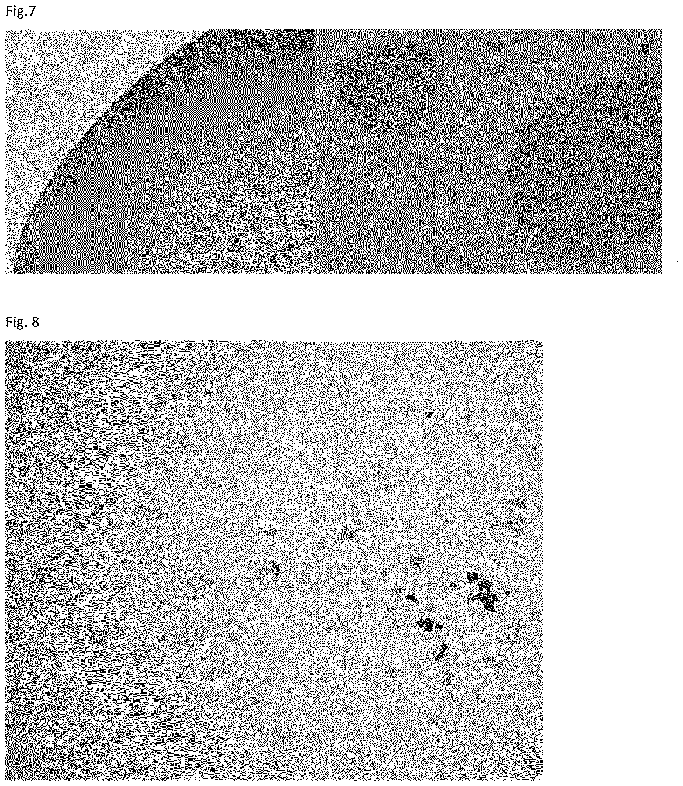

Fig. 7A, shows droplets of various sizes at the outskirts of the CaCl₂ droplet, while in Fig. 7B, it is shown droplets of relatively monodisperse size in the center of the droplet There seems to be some coalescence due to the mechanical stress of pipetting and deposition in the droplet. Not all the emulsion pockets in the droplets were as polydisperse as in fig. 7A.

### Droplets post-processing:

### Same day post-processing

After addition of the PFO, non-spherical particles of various size are shown in Fig. 8. This tends to indicate that the solidification was fast and occurred upon contact with the CaCl₂. Small emulsions could also be observed. Osmosis, due to the difference in concentration between the water droplet and the emulsion might have led to smaller droplets which were not destabilized by the PFO.

### Post-processing after overnight incubation in "wetbox"

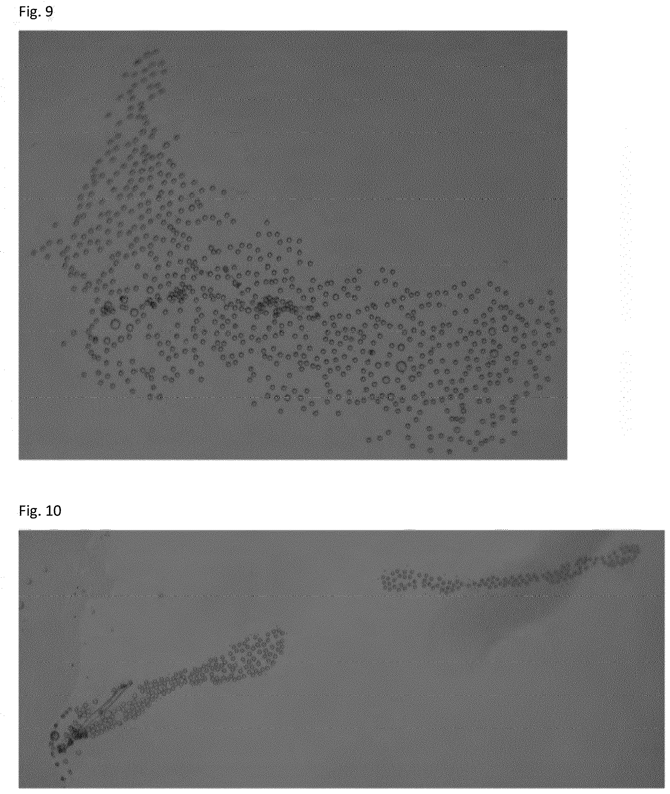

Fig. 9 shows that the droplets have shrunk overnight compared to after deposition in the CaCl₂ droplet (Fig. 7). The difference in CaCl₂ concentration between the droplets and surrounding aqueous phase resulted in osmosis leading to shrinking of the emulsions.

The alginate solution was prepared in MilliQ. Using a buffer solution such as 1X PBS (1.05 mM KH₂PO₄, 155 mM NaCl, 3 mM Na₂HPO₄-7(H₂O)) when preparing the alginate solution might counteract the osmosis effect. Solidification will be used to encapsulate cells, therefore media will be used instead of MilliQ. The media might contain enough salts to counteract the osmosis effect.

After addition of PFO, no change can be observed (fig. 9 vs 10) whereas a change can be observed after addition of PFO without overnight incubation (fig. 7 vs 8). This tends to indicate that the droplets were already broken, resuspended in water and therefore solidified.

The resulting particles are relatively monodisperse, spherical and homogeneous. These properties indicate that the solidification was a slow process and therefore solidification of alginate via micellar transport was successful.

### Solidification of alginate emulsions in bulk

Compared to the "wetbox" experiment, in bulk, the alginate droplets did not shrink as much in the bulk experiment (fig. 9 vs 11). This could be because all the droplets in the "wetbox" experiment were in close contact with the aqueous phase containing 200 mM CaCl₂ whereas in the bulk experiment most of emulsions were not in close proximity to the interface of 200 mM CaCl₂ and HFE7500.

Disparity in size can be observed between the different droplet which could be explained by the distance the emulsions are from the interface of HFE7500 and 200 mM CaCl₂. The closer the droplet are from the surface the easier osmosis can occur. The size difference could also be explained by droplet size disparity during droplet making or coalescence.

After addition of PFO, the alginate did not diffuse into the aqueous phase, the resulting particles are spherical and homogeneous. These properties indicate that the solidification was a slow process and therefore solidification of alginate via micellar transport was successful.

### Example 4:

### 2-layer alginate beads

An alginate solution was cut in a microfluidic flow focusing device with oil containing an anionic surfactant to produce alginate in oil emulsions (10 % w/w anionic surfactant in oil). The emulsions were collected in a 1.5 mL microfuge tube and added to a dynamic droplet incubator. The dynamic droplet incubator allowed the cycling of the calcium solubilized in the oil by the anionic surfactant, and therefore a better diffusion of the calcium to the droplets. After 2 hours incubation the droplets were solidified becoming beads. The beads were resuspended in an aqueous solution using the droplet breaker as previously described and the oil was removed.

In one embodiment, the droplets were solidified and becoming beads after 1 min to 24 h. In a preferred embodiment the droplets were solidified and becoming beads after 5 min to 12 h. In a more preferred embodiment the droplets were solidified and becoming beads after 30 min to 6 h. In an even more preferred embodiment the droplets were solidified and becoming beads after 1h to 4 h and most preferably after 2h.

The aqueous suspension solution was then removed and the beads resuspended in an alginate solution. The beads in alginate solution was cut in a microfluidic flow focusing device with oil containing an anionic surfactant to produce beads in alginate in oil emulsions. The emulsions were collected in a 1.5 mL microfuge tube and added to a dynamic droplet incubator for solidification as previously described.

The 2-layer alginate beads were then resuspended in buffer aqueous solution using the droplet breaker as previously described and the oil was removed. The geometry of the produced particles was determined under a confocal microscope which showed a 2-layered alginate capsule.

### Example 5

### Viability comparison of cells encapsulated in alginates solidified via acid dechelation or micellar transport of calcium

A cell line, sampled from the gut was grown in recommended medium until 90% confluency in a flask. The cells were detached from the surface of flask using trypsin-EDTA (Ethylenediaminetetraacetic acid) and resuspended in two separate solutions. A solution containing medium and alginate and a second containing medium, alginate and divalent ions complexed with a chelating agent (Ca-EDTA).

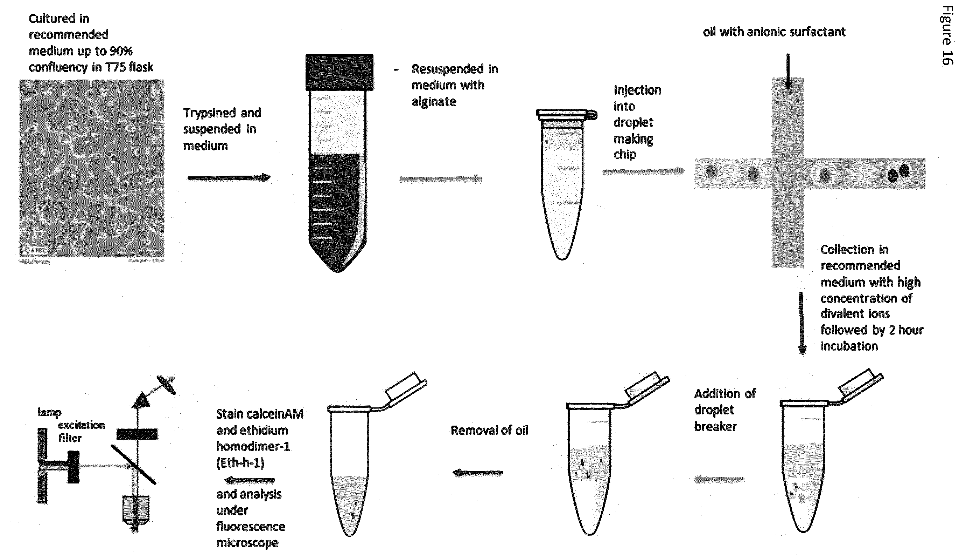

The solution containing only cells and alginate was cut in a microfluidic flow-focusing device with oil containing an anionic surfactant to produce alginate in oil emulsions. The emulsions were collected in medium containing a high concentration of calcium and incubated 2 hours before being resuspended in the aqueous phase using the droplet breaker as previously described. The oil was removed and the percentage viability was assessed by incubating the beads in medium containing calceinAM and ethidium homodimer-1 for 20 min followed by observation under the fluorescence microscope (fig. 16).

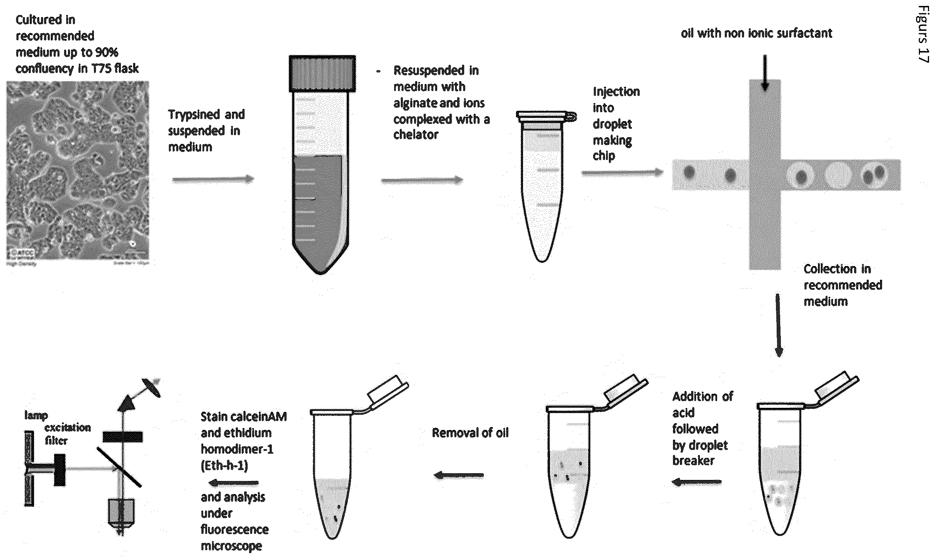

The solution containing cells, alginate and chelated divalent ions was cut in a microfluidic flow focusing device with oil containing a nonionic surfactant to produce alginate in oil emulsions. The emulsions were collected in medium, acid was added and briefly incubated. The beads were resuspended in the aqueous phase using the droplet breaker as previously described. The oil was removed and the percentage viability was assessed by incubating the beads in medium containing calceinAM and ethidium homodimer-1 for 20 min followed by observation under the fluorescence microscope (fig. 17). The results were compared to the viability of the cells encapsulated in alginate using micellar transport.

The comparison of both results showed an improvement in viability using micellar transport compared to releasing divalent ions from a chelating agent using acid.

The methods according to the present invention can find different applications:
- Isolation of a chemicals, enzymes, proteins, antibodies, polymers, cells, microorganisms, etc. individually within a sample for analysis of all the chemicals, enzymes, proteins, antibodies, polymers, cells, microorganisms, etc. individually via, for example, droplet microfluidics or flow cytometry instruments.
- Analysis and sorting of isolated chemicals, enzymes, proteins, antibodies, polymers, cells, microorganisms, etc. or a combination of chemicals, enzymes, proteins, antibodies, polymers, cells, microorganisms, etc. in alginate beads via a flow sorting instrument such as FACS or COPAS into individual wells of a microtiter plate.
- Protection of microorganisms such as probiotics or drugs against pathogens or acidic environments when ingested by individuals.
- Protection of encapsulated cells or microorganisms in hot but more importantly cold environments such as when freezing for storage.
- Support for the capture on its surface of molecules such as antibodies in an ELISA bead assay or nucleic acids in a DNA extraction protocol. One of the advantages being alginate is biocompatible compared to commonly used magnetic beads.

### Brief description of the figures

Figure 1: Images of the design for the flow-focusing microfluidic device (FFD)
Figure 2: Images of the alginate in HFE7500 emulsions deposited in 200 mM CaCl₂ droplets observed under the bright field microscope of the fluorescence microscope at t=0 (20X objective)
Figure 3: Images of the alginate in HFE7500 emulsions deposited in 200 mM CaCl₂ droplets, incubated at room temperature for 1h, observed under the bright field microscope of the fluorescence microscope, before (A) and after (B) addition of PFO (20X objective)

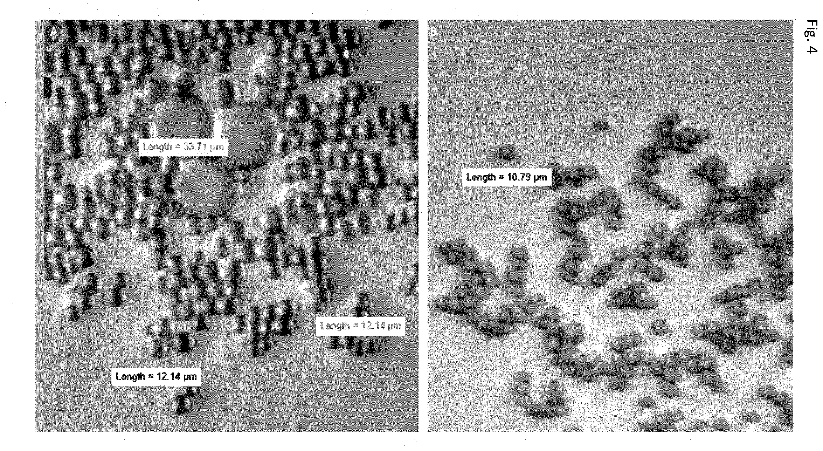

Figure 4: Images of the alginate in HFE7500 emulsions deposited in 200 mM CaCl₂ droplets, incubated at room temperature for 2h, observed under the bright field microscope of the fluorescence microscope, before (A) and after (B) addition of PFO (20X objective)
Figure 5: Images of the alginate in HFE7500 emulsions deposited in 200 mM CaCl₂ droplets, incubated at room temperature for 4h, observed under the bright field microscope of the fluorescence microscope, before (A) and after (B) addition of PFO (20X objective)
Figure 6: Images of the alginate in HFE7500 emulsions deposited in 200 mM CaCl₂ droplets, incubated at room temperature for 24h, observed under the bright field microscope of the fluorescence microscope, before (A) and after (B) addition of PFO (20X objective)
Figure 7: Images of the alginate in HFE7500 emulsions deposited in 200 mM CaCl₂ droplets observed under the bright field microscope of the fluorescence microscope, 4X objective
Figure 8: Images of the alginate in HFE7500 emulsions deposited in 200 mM CaCl₂ droplets, after addition of PFO, observed under the bright field microscope of the fluorescence microscope (4X objective)
Figure 9: Images of the alginate in HFE7500 emulsions deposited in 200 mM CaCl₂ droplets, incubated at room temperature overnight, observed under the bright field microscope of the fluorescence microscope (4X objective)
Figure 10: Images of the alginate in HFE7500 emulsions deposited in 200 mM CaCl₂ droplets, incubated at room temperature overnight, after addition of PFO observed under the bright field microscope of the fluorescence microscope (4X objective)

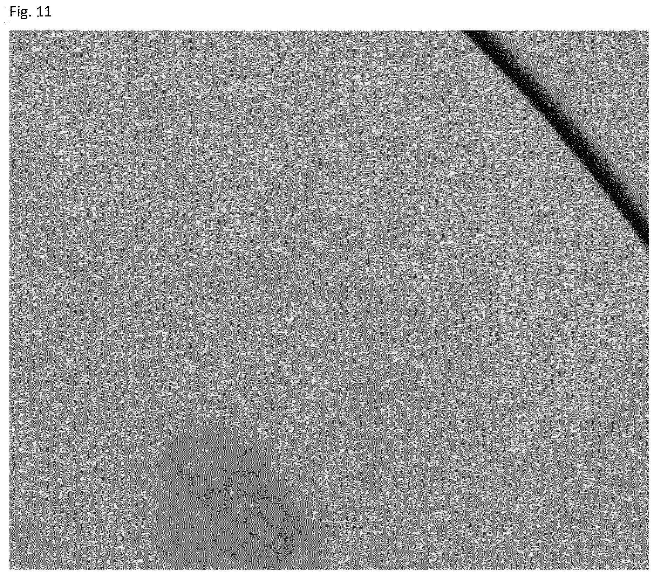

Figure 11: Images of the alginate in HFE7500 emulsions from the collection tube, incubated at room temperature overnight, observed under the bright field microscope of the fluorescence microscope (10X objective)

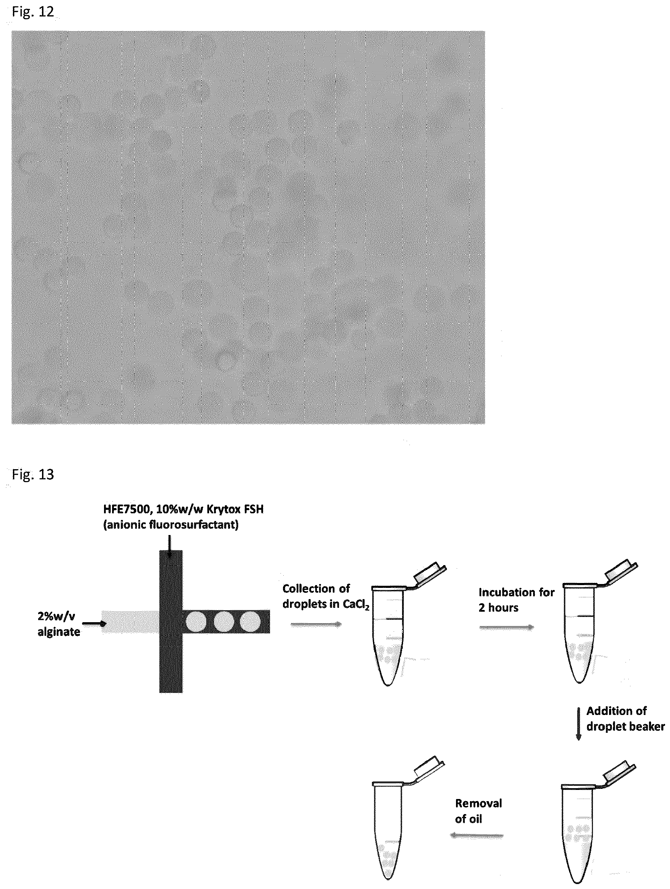

Figure 12: Images of the alginate in HFE7500 emulsions from the collection tube, incubated at room temperature overnight, after addition of PFO observed under the bright field microscope of the fluorescence microscope (10X objective)
Fig. 13: Diagram of the workflow for the production of alginate droplets and solidification via micellar transport of Ca²⁺.

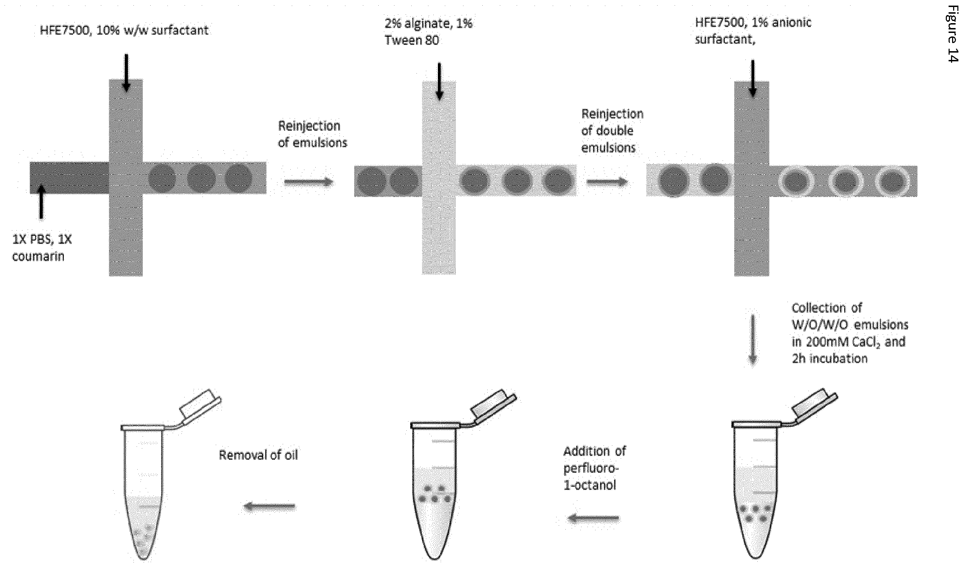

Fig. 14: Diagram of the workflow for the production of triple emulsions and solidification via micellar transport of Ca²⁺.

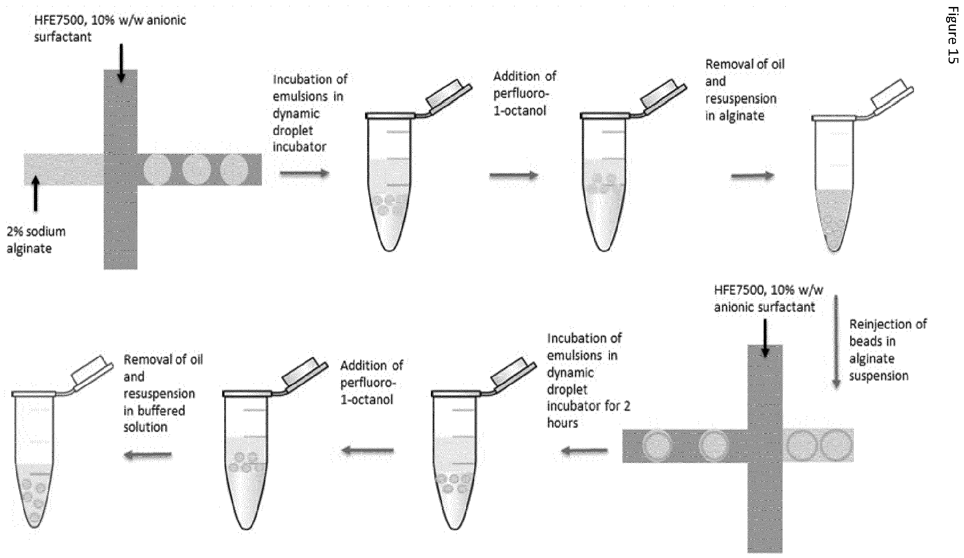

Fig. 15: Diagram of the workflow for the production of 2-layer alginate droplets and solidification via micellar transport of Ca²⁺.
Fig. 16: Diagram of the workflow for the viability assay of the encapsulation of epithelial cells in alginate beads via micellar transport of divalent ions
Fig. 17: Diagram of the workflow for the viability assay of the encapsulation of epithelial cells in alginate beads via acid dechelation of divalent ions.

## Claims

1. A method for producing alginate beads, wherein the alginate solidifies via slow release of divalent ions into an alginate solution, comprising the steps of:
- preparing a first solution containing alginate
- preparing a second solution containing an anionic surfactant in oil,
- preparing a third solution containing divalent ions
- injecting said first and second solutions into a microfluidic device generating alginate in oil droplets
- collecting and incubating the alginate in oil droplets in the third solution, inducing solidification of the first solution.

2. The method according to claim 1, wherein the first solution comprises sodium alginate at 2% w/v.

3. The method according to claims 1-2, wherein the second solution comprises a fluorinated oil Krytox FSH.

4. The method according to claims 1-3, wherein the divalent ions are Ca²⁺or Ba²⁺.

5. The method according to claims 1-4, wherein to the alginate in oil emulsion in the third solution is added a droplet breaker,

6. the method according to claims 1-5, wherein the droplet breaker is 1H,1H,2H,2H-Perfluoro-1-octanol.

7. The method according to claims 1-5, wherein the solidification of the first solution is done via micellar transport of divalent ions from the third solution into the first solution via the second solution.

8. The method according to claims 1-7, wherein the first solution comprises sodium alginate in cell culture medium and wherein cells are resuspended in said first solution.

9. A method for producing hollow alginate beads, wherein the alginate solidifies via slow release of divalent ions into an alginate solution, comprising the steps of:
- preparing a first solution containing alginate, consisting of water in oil in alginate double emulsions;
- preparing a second solution containing an anionic surfactant in oil;
- preparing a third solution containing divalent ions;
- injecting said first and second solutions into a microfluidic device generating alginate in oil emulsions;
- collecting and incubating the alginate in oil droplets in the third solution, inducing solidification of the first solution.

10. Use of the method according to claims 1-8 for co-culturing of fluorescent reporter cell-lines and microorganisms.

11. The use according to claim 10, wherein the fluorescent reporter cell-line is encapsulated in the hollow alginate bead.

12. The use according to claims 10-11, wherein the microorganism is encapsulated in the hollow alginate bead.

13. The use according to claims 10-12, wherein the microorganism is tested for activity on the fluorescent reporter cell line

14. The use according to claims 10-13, wherein the fluorescent reporter cell-line is analyzed for a change in fluorescence.

15. A method for producing 2-layer alginate beads, wherein the alginate solidifies via slow release of divalent ions into an alginate solution, comprising the steps of:
- preparing a first solution containing alginate, consisting consists of a suspension of alginate beads in a alginate containing solution;
- preparing a second solution containing an anionic surfactant in oil;
- preparing a third solution containing divalent ions;
- injecting said first and second solutions into a microfluidic device generating alginate in oil emulsions;
- collecting and incubating the alginate in oil droplets in the third solution, inducing solidification of the first solution.
